# EUROPEAN PATENT APPLICATION

(11) **EP 4 844 391 A1**
(43) Date of publication of application: **25.11.2026**
(21) Application number: 26177997.9
(22) Date of filing: 11.05.2026
(51) Int. Cl.: G01N 27/327, C12Q 1/6825, G01N 33/487

(54) **METHODS AND SYSTEMS FOR CONTROLLING MOLECULES IN MICRO-ELECTRO-MECHANICAL SYSTEMS SENSORS**

(30) Priority: 19.05.2025 US 202519212216
(71) Applicant: Robert Bosch GmbH, 70442 Stuttgart (DE)
(72) Inventor: Kornbluth, Mordechai, Brighton, Massachusetts, 02135 (US); Gadelrab, Karim, Boston, MA, 02111 (US); Darvish, Armin, Brisbane, CA, 94005 (US)

(57) **Abstract**

Systems and methods for controlling the proximity of a biomolecule to an electrode. Systems may include a device for sensing a biomolecule including at least one electrode, a channel, a voltage source, a processor, a memory coupled to the processor, storing executable instructions, and a controller. Electrode(s) may be coated with a surface coating that may modulate a distance of a biomolecule from the electrode(s). Methods may include providing a device including at least one electrode and a channel and providing a biomolecule and a solvent to the channel of the device. Methods may also include modulating the distance between the biomolecule and the electrode by coating the at least one electrode with a surface coating; modifying the surface charge of the at least one electrode; and/or applying a dynamic voltage to the at least one electrode to dynamically modulate the distance of the biomolecule from the electrode.

## Description

### TECHNICAL FIELD

The present disclosure relates to methods and systems for controlling molecules (e.g., biomolecules) in micro-electro-mechanical systems (MEMS) sensors.

### BACKGROUND

Biosensor Micro-Electro-Mechanical Systems (MEMS) may provide highly sensitive and accurate means for detecting and analyzing biomolecules. Biosensor MEMS may use nanochannels to manipulate and move biomolecules in solution. These nanochannels may allow for precise control and manipulation of biomolecules and may enable them to be positioned next to nanoelectrodes for measurement. Control of the proximity between the biomolecule and the electrode may be challenging. Ensuring that the biomolecule is positioned at the optimal distance from the nanoelectrode is important for accurate measurements.

### SUMMARY

In at least an aspect, a system for controlling the proximity of a biomolecule to an electrode is provided. The system may comprise a device for sensing a biomolecule including at least one electrode, wherein the at least one electrode is coated with a conductive low-van der Waals material to form a surface coating, and a channel sized to receive the biomolecule and a solvent, wherein the surface coating of the electrode is configured to modulate a distance of the biomolecule from the at least one electrode in the channel. The system may additionally include a voltage source and a processor, a memory coupled to the processor and storing executable instructions, and a controller. The executable instructions, when executed by the processor, may cause the controller to transmit a signal to the at least one electrode to sense the biomolecule.

In at least another aspect, a system for controlling the proximity of a biomolecule to an electrode is provided. The system may comprise a device for sensing a biomolecule including at least one electrode and a channel sized to receive the biomolecule and a solvent; a voltage source; and a processor, a memory coupled to the processor and storing executable instructions, and a controller. The executable instructions, when executed by the processor, may cause the controller to transmit a signal to the at least one electrode to charge the at least one electrode with a surface charge between -0.5 and +0.5 C/m2. The surface charge may modulate a distance of the biomolecule from the at least one electrode.

In yet another aspect, a system for controlling the proximity of a biomolecule to an electrode is provided. The system may include a device for sensing a biomolecule including at least one electrode and a channel sized to receive the biomolecule and a solvent; a voltage source; and a processor, a memory coupled to the processor and storing executable instructions, and a controller. The executable instructions, when executed by the processor, may cause the controller to transmit a signal to the at least one electrode to dynamically modify a voltage of the at least one electrode by a pulse of length τ that is substantially different from the electrode sensing time τ' such that τ/τ^' is less than 0.1 or greater than 10.

In yet another aspect, a method for controlling the proximity of a biomolecule to an electrode is provided. The method may comprise providing a device including at least one electrode and a channel; providing a biomolecule and a solvent to the channel of the device; and modulating a distance of the biomolecule from the at least one electrode by one of: coating the at least one electrode with a surface coating prior to providing the biomolecule and the solvent to the channel; applying a voltage to the at least one electrode to modify the surface charge of the electrode; or dynamically modifying a voltage of the at least one electrode.

### BRIEF DESCRIPTION OF THE DRAWINGS

Figure 1 is a graph illustrating the effects of proximity on signals.
Figure 2A and 2B illustrate an exemplar device including a nanochannel, coating, and electrode (e.g., a patterned electrode).
Figure 3 illustrates an exemplar system for controlling the proximity of a biomolecule to an electrode according to an embodiment.
Figure 4A illustrates an exemplar device having a single adjustable voltage difference.
Figure 4B illustrates an exemplar device having a single AC voltage difference.
Figure 5 illustrates an exemplar device having multiple voltages relative to ground.
Figure 6 illustrates an exemplar device having multiple voltages adjusted by resistors.
Figures 7A and 7B are graphs illustrating data from a simulation of the interaction of a DNA molecule with a metal electrode using a molecular dynamics simulation.
Figure 7C is an image of the interaction of the DNA with the metal electrode using the molecular dynamics simulation.
Figures 8A, 8B, and 8C are graphs illustrating the free energy of interaction at different salt concentrations and the distance between the DNA center and the electrode surface.
Figure 9 is a graph illustrating the dependencies of free energy of interaction on different surface charging states at a fixed salt concentration.

### DETAILED DESCRIPTION

As required, detailed embodiments of the present disclosure are disclosed herein; however, it is to be understood that the disclosed embodiments are merely exemplary of the disclosure that may be embodied in various and alternative forms. The figures are not necessarily to scale; some features may be exaggerated or minimized to show details of particular components. Therefore, specific structural and functional details disclosed herein are not to be interpreted as limiting, but merely as a representative basis for teaching one skilled in the art to variously employ the present disclosure.

Except in the examples, or where otherwise expressly indicated, all numerical quantities in this description indicating amounts of material or conditions of reaction and/or use are to be understood as modified by the word "about". The first definition of an acronym or other abbreviation applies to all subsequent uses herein of the same abbreviation and applies mutatis mutandis to normal grammatical variations of the initially defined abbreviation; and, unless expressly stated to the contrary, measurement of a property is determined by the same technique as previously or later referenced for the same property.

Unless indicated otherwise, all technical and scientific terms used herein have the same meaning as commonly understood by one of ordinary skill in the art to which the present disclosure belongs.

This disclosure is not limited to the specific embodiments and methods described below, as specific components and/or conditions may, of course, vary. Furthermore, the terminology used herein is used only for describing particular embodiments and is not intended to be limiting in any way.

As used in the specification and the appended claims, the singular form "a," "an," and "the" comprise plural referents unless the context clearly indicates otherwise. For example, reference to a component in the singular is intended to comprise a plurality of components.

The terms "or" and "and" can be used interchangeably and can be understood to mean "and/or".

The term "comprising" is synonymous with "including," "having," "containing," or "characterized by." These terms are inclusive and open-ended and do not exclude additional, unrecited elements or method steps.

The phrase "consisting of" excludes any element, step, or ingredient not specified in the claim. When this phrase appears in a clause of the body of a claim, rather than immediately following the preamble, it limits only the element set forth in that clause; other elements are not excluded from the claim as a whole.

The phrase "consisting essentially of" limits the scope of a claim to the specified materials or steps, plus those that do not materially affect the basic and novel characteristic(s) of the claimed subject matter.

The terms "comprising", "consisting of", and "consisting essentially of" can be alternatively used. When one of these three terms is used, the presently disclosed and claimed subject matter can include the use of either of the other two terms.

Integer ranges may explicitly include all intervening integers. For example, the integer range 1-10 explicitly includes 1, 2, 3, 4, 5, 6, 7, 8, 9, and 10. Similarly, the range 1 to 100 includes 1, 2, 3, 4. . . . 97, 98, 99, 100. Similarly, when any range is called for, intervening numbers that are increments of the difference between the upper limit and the lower limit divided by 10 can be taken as alternative upper or lower limits. For example, if the range is 1.1. to 2.1 the following numbers 1.2, 1.3, 1.4, 1.5, 1.6, 1.7, 1.8, 1.9, and 2.0 can be selected as lower or upper limits. In the specific examples set forth herein, concentrations, temperature, and reaction conditions (e.g., pressure, pH, etc.) can be practiced with plus or minus 50 percent of the values indicated rounded to three significant figures. In a refinement, concentrations, temperature, and reaction conditions (e.g., pressure, pH, etc.) can be practiced with plus or minus 30 percent of the values indicated rounded to three significant figures of the value provided in the examples. In another refinement, concentrations, temperature, and reaction conditions (e.g., pH, etc.) can be practiced with plus or minus 10 percent of the values indicated rounded to three significant figures of the value provided in the examples.

In any examples set forth herein, concentrations, temperature, and reaction conditions (e.g., pressure, pH, flow rates, etc.) can be practiced with plus or minus 50 percent of the values indicated rounded to or truncated to two significant figures of the value provided in the examples. In a refinement, concentrations, temperature, and reaction conditions (e.g., pressure, pH, flow rates, etc.) can be practiced with plus or minus 30 percent of the values indicated rounded to or truncated to two significant figures of the value provided in the examples. In another refinement, concentrations, temperature, and reaction conditions (e.g., pressure, pH, flow rates, etc.) can be practiced with plus or minus 10 percent of the values indicated rounded to or truncated to two significant figures of the value provided in the examples.

The terms "polynucleotide", "nucleotide", "nucleotide sequence", "nucleic acid", "polynucleic acid", and "oligonucleotide" may be used interchangeably in one or more embodiments this disclosure. They refer to a polymeric form of nucleotides of any length, either deoxyribonucleotides or ribonucleotides, or analogs thereof. Polynucleotides may have any three-dimensional structure, and may perform any function, known or unknown. The following are non-limiting examples of polynucleotides: single-, double-, or multi-stranded DNA or RNA, genomic DNA, cDNA, DNA -RNA hybrids, or a polymer comprising purine and pyrimidine bases or other natural, chemically or biochemically modified, non-natural, or derivatized nucleotide bases. The terms "polynucleotide" and "nucleic acid" may include, as applicable to the embodiment being described, single-stranded (such as sense or antisense) and double-stranded polynucleotides. A polynucleotide may comprise one or more modified nucleotides, such as methylated nucleotides and nucleotide analogs. A polynucleotide may additionally comprise one or more nucleotides modified with a labeling molecule. If present, modifications to the nucleotide structure may be imparted before or after assembly of the polymer. The sequence of nucleotides may be interrupted by non-nucleotide components. A polynucleotide may be further modified after polymerization, such as by conjugation with a labeling component. The term "base" refers to a single nucleotide in one or more embodiments.

The terms "complementarity" or "complement" may refer to the ability of a nucleic acid to form hydrogen bond(s) with another nucleic acid sequence by either traditional Watson-Crick or other non-traditional types. A percent complementarity indicates the percentage of residues in a nucleic acid molecule which can form hydrogen bonds (e.g., Watson-Crick base pairing) with a second nucleic acid sequence (e.g., 4, 5, and 6 out of 6 being 66.67%, 83.33%, and 100% complementary). "Perfectly complementary" means that all the contiguous residues of a nucleic acid sequence will hydrogen bond with the same number of contiguous residues in a second nucleic acid sequence. "Substantially complementary" as used herein refers to a degree of complementarity that is at least 40%, 50%, 60%, 62.5%, 70%, 75%, 80%, 85%, 90%, 95%, 97%, 98%, 99%, or 100%, or percentages in between over a region of 4, 5, 6, 7, and 8 nucleotides, or refers to two nucleic acids that hybridize under stringent conditions.

The processes, methods, or algorithms disclosed herein can be deliverable to and/or implemented by a processing device, controller, or computer, which can include any existing programmable electronic control unit or dedicated electronic control unit. Similarly, the processes, methods, or algorithms can be stored as data and instructions executable by a controller or computer in many forms including, but not limited to, information permanently stored on non-writable storage media such as ROM devices and information alterably stored on writeable storage media such as floppy disks, magnetic tapes, CDs, RAM devices, and other magnetic and optical media. The processes, methods, or algorithms can also be implemented in an executable software object. Alternatively, the processes, methods, or algorithms can be embodied in whole or in part using suitable hardware components, such as Application Specific Integrated Circuits (ASICs), Field-Programmable Gate Arrays (FPGAs), state machines, controllers or other hardware components or devices, or a combination of hardware, software and firmware components.

Biosensor Micro-Electro-Mechanical Systems (MEMS) are used in medical diagnostics and biological research by providing a highly sensitive and accurate means of detecting and analyzing biomolecules. One of the components of biosensor MEMS are nanochannels, which are tiny channels on the order of dozen to hundreds of nanometers in size, to manipulate and move biomolecules such as DNA, often within water. These nanochannels allow for precise control and manipulation of biomolecules, enabling them to be positioned next to nanoelectrodes for measurement.

The interaction between the biomolecule and the nanoelectrode is then measured by the flow of current, providing valuable information about the biomolecule's properties and behavior. This technology has wide-ranging applications in fields such as medical diagnostics, drug development, and genetic research, where the ability to accurately measure and analyze biomolecules is crucial. The use of nanochannels in biosensor MEMS has opened new possibilities for highly sensitive and precise detection and analysis of biomolecules.

A challenge in biosensor MEMS utilizing nanochannels to move biomolecules next to nanoelectrodes is the precise control of the proximity between the biomolecule and the electrode. Ensuring that the biomolecule is positioned at the optimal distance from the nanoelectrode is crucial for accurate measurements of the interactions between the two. There is thus a need for systems and methods for enhancing the sensitivity and reliability of biosensor MEMS in detecting and analyzing biomolecular interactions.

If a biomolecule is placed in a sensing device including an electrode of a width at least 10 nm and a nanochannel of width 10 to 1000 nm, for example, the biomolecule may pass within a particular distance to the electrode within the nanochannel. The distance between the biomolecule and the electrode may be a distance z. If z is increased, there will be less friction (on a nanoscale sense) between the biomolecule and the electrode. The reduced friction may enable a higher velocity and decrease the stickiness of the biomolecule to the electrode. If z is decreased, the sensing mechanism is magnified, thereby increasing the signal and the signal to noise ratio (SNR). Carefully controlling the distance between the biomolecule and the electrode optimizes the signal to noise ratio and allows for control over whether and how strongly the biomolecule sticks to the electrode.

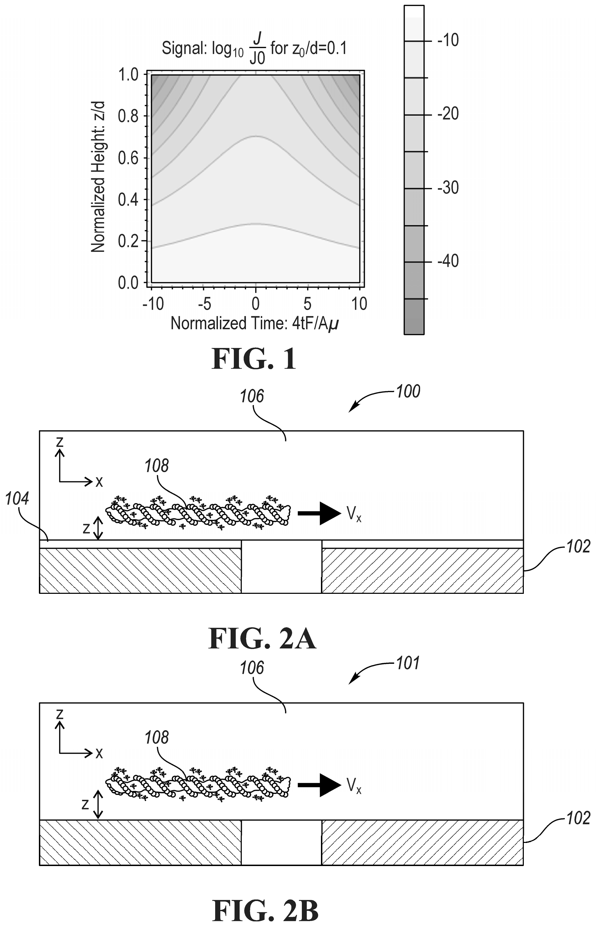

Figure 1 is a graph illustrating the effects of proximity on signals. In an ideal scenario, a biomolecule with velocity vx passing by an electrode of sensing zone d at height zm may generate a signal of a Gaussian lineshape of the following form, where t is time and z0 and J0 are both parameters. $j \left(t\right) = {J}_{0} exp \left(-\frac{2}{{z}_{0}}\sqrt{{d}^{2} + {z}_{m}^{2}}\right) exp \left(-\frac{16 {t}^{2} {v}_{x}^{2}}{{d}^{2}}\right)$

The prefactor's exponential dependence on zm means that for zm~d it will be critical to control the height zm for an appropriate signal magnitude.

Simultaneously, the width of the peak is proportional to d/vx, lending to velocity control, which can be given by an effective viscosity µ, which relates the velocity to the applied force as follows: vx=Fzm/µA, where F is the force of the fluid on the biomolecule, µ is the effective viscosity, and A is the effective area. Converting to dimensionless units and assuming a value of z¬0/d=0.1, gives the plot in Figure 1.

Systems and methods for controlling the proximity of a biomolecule to an electrode are provided herein in one or more embodiments. The systems and methods may include a device used to sense a biomolecule (e.g., the device may include at least one electrode) and methods for modulating the surface charge, salt concentration, voltage, and/or surface coating of the electrode, to modify the proximity of the biomolecule to the electrode, thereby controlling both the signal measured and the velocity (e.g., signal width and flow rate).

In one or more embodiments, a system for controlling the proximity of a biomolecule to an electrode is provided. The system may include a device and a voltage source. The device may be used to sense a biomolecule. The device may be a biomolecule MEMS sensor. The biomolecule MEMS sensor may include at least one electrode. The electrode may be a platinum electrode. Additional non-limiting potential electrode materials may include carbon, graphite, gold, silver, silicon, aluminum, tungsten, rhodium ruthenium, palladium, titanium, or any combination thereof. The electrode may be coated by a surface coating. The surface coating may be a low-van der Waals material (e.g., a material having an exfoliation energy of below 150 meV/Å2 and/or below 300 meV/atom )). The low-van der Waals material may be a conductive material. The surface coating may be at least one layer of carbon or graphene. In an example, a platinum electrode may be coated with a monolayer of carbon, such as graphene, or a plurality of layers of carbon. The surface coating may alternatively be an MXene. MXenes are highly conductive 2D materials, typically following the formula Mn+1XnTx, where M is an early transition metal (e.g., titanium, vanadium or molybdenum), X is carbon and/or nitrogen, T is a functional group (e.g., -OH, -F, -Cl, or =O), and n can vary from 1 to 4. For example, the surface coating may be Ti₃C₂Tₓ. Ti₃C₂Tₓ has demonstrated conductivity up to 1.5×104 S/cm). The surface coating may alternatively be Nb₄C₃Tₓ (niobium carbide MXene). The multi-layered structure (n=3) of niobium carbide MXene yields lower resistivity with single-layer Nb₄C₃Tₓ flakes showing an in-plane conductivity of ~1024 S/cm. Surface functionalization T is an experimental tuning parameter to control conductivity. The surface coating on the electrode may modulate whether and how strongly a biomolecule sticks to the electrode.

In one or more embodiments, the electrode may be platinum or metal. The platinum or metal electrode may be deposited precisely using atomic layer deposition (ALD) for example, thus shielding the van der Waals interaction of platinum. In other embodiments, a carbon coating may be deposited with solvated graphene or graphene oxide flakes, for example, followed by a drying (solvent evaporation) step.

The biomolecule MEMS sensor may additionally include a channel. The channel may be a nanochannel having at least one dimension of < 1 µm. For example, the channel may be a nanochannel with a width of < 1 µm or with a diameter of < 1 µm. The channel may be sized to receive a solvent and one or more biomolecules. The solvent may include water and salts, and the channel may be sized to receive a DNA molecule, for example. Alternatively, the channel may be a micro or millimeter-sized channel.

The system may further include a voltage source. The voltage source may interact with the at least one electrode of the device. For example, the voltage source may be used to apply a voltage to the at least one electrode of the device to modify the surface charge of the electrode. The voltage applied to the at least one electrode of the device may be static or dynamic. The charge on the electrode may be modulated dynamically to adjust the proximity between a biomolecule and the electrode. For example, applying a dynamic voltage to the electrode may cause the biomolecule to stick to the electrode for a sufficient time to collect a measurement. The voltage may then be changed to unstick the biomolecule and allow flow to move the biomolecule. Alternatively, the voltage fluctuation can be much faster than the measurement time, allowing an averaging effect to resist or avoid contamination of the measurement.

In one or more embodiments, the biomolecule MEMS sensor may include two or more electrodes. The voltage source may be used to control the charges on each electrode either dynamically or statically to ensure a desired flow rate. The two or more electrodes may be patterned in a +/-/+/- alternation. As disclosed in Curk et al. (Phys. Rev. Lett. 133, 078201, doi: 10.1103/PhysRevLett.133.078201), charges on each electrode are able to control ion flow as shown using dissipative particle dynamics (DPD) simulations.

In one or more embodiments, the biomolecule MEMS sensor may include more than one electrode. Insulating (e.g., dielectric) barriers may be included between electrodes. Each of the electrodes may be held at different potentials. Where electrodes are held at different potentials, there should not be conductive coating on top of the insulating (e.g., dielectric) barriers between the electrodes, or there may be shorting to the electrodes. Several techniques may be used to ensure that there is no conductive coating on top of the insulating (e.g., dielectric) barriers between the electrodes, including (a) voltage-induced deposition where the coating is preferentially deposited on high- or low-voltage domains, which can be applied specifically to the electrodes and not the insulating barriers; or (b) applying a mask on the insulating barriers before a fluid-based deposition method, followed by a cleaning stage to remove the mask and/or coating from the barriers.

Figure 2A illustrates a device 100 according to an embodiment. The device 100 may be a biomolecule MEMS sensor. The device 100 may be part of a system for controlling the proximity of a biomolecule to an electrode according to an embodiment. The device 100 may include at least one electrode 102. The at least one electrode 102 may be patterned. The at least one electrode 102 may be a platinum electrode. The at least one electrode 102 may be coated with a surface coating 104. The surface coating 104 may be a conductive low-van der Waals material. The device 100 may further include a channel 106. The channel 106 may be a nanochannel. The channel 106 may be sized to receive a biomolecule 108 and a solvent (not shown). The biomolecule 108 may be a DNA molecule, also referred to as a DNA strand. The biomolecule may be traveling through the channel 106 at a velocity vx and be positioned at a distance z from the at least one electrode 102.

Figure 2B illustrates a device 100 according to an embodiment. The device 100 may be a biomolecule MEMS sensor. The device 100 may be part of a system for controlling the proximity of a biomolecule to an electrode according to an embodiment. The device 100 may include at least one electrode 102. The at least one electrode 102 may be patterned. The at least one electrode may be a graphite electrode. The device 100 may further include a channel 106. The channel 106 may be a nanochannel. The channel 106 may be sized to receive a biomolecule 108 and a solvent (not shown). The biomolecule 108 may be a DNA molecule, also referred to as a DNA strand. The biomolecule may be traveling through the channel 106 at a velocity vx and be positioned at a distance z from the at least one electrode 102.

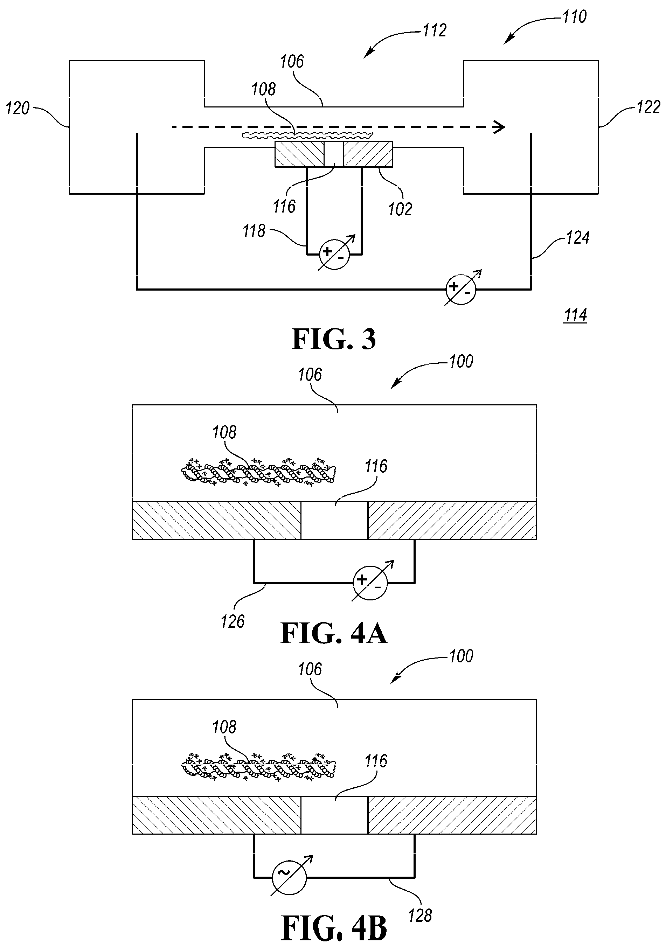

Figure 3 illustrates a system 110 for controlling the proximity of a biomolecule to an electrode according to an embodiment. The system 110 may include a device 112 and a voltage source 114 (not shown). The device 110 may include at least one electrode 102. The at least one electrode 102 may be a platinum or metal electrode. The at least one electrode 102 may be a carbon or graphite electrode. The at least one electrode 102 may be coated with a surface coating (not shown). The surface coating may be a conductive low-van der Waals material. The device 110 may further include a sensing component 116. The sensing component 116 may be formed from two electrodes separated by a dielectric. The sensing component 116 may have a distinct voltage 118 applied to it. The device 110 may further include a channel 106. The channel 106 may be a nanochannel. The channel 106 may be sized to receive a biomolecule 108 and a solvent (not shown). The biomolecule 108 may be a DNA molecule, also referred to as a DNA strand. The device 110 may further include a source basin 120 and a drain basin 122. The source basin 120 and the drain basin 122 may each be in fluidic communication with the channel 106. The source basin 120 and the drain basin 122 may each be in electrical communication with an electrode. The source basin 120 and the drain basin 122 may each be subject to an electrophoretic driving voltage 124.

Figure 4A illustrates a device 100 having a single adjustable voltage difference 126. The single adjustable voltage difference 126 may facilitate setting the voltage output to a desired level within a range. The single adjustable voltage difference 126 may interact with the sensing component 116 and facilitate sensing of the biomolecule 108 in the channel 106.

Figure 4B illustrates a device 100 having a single AC voltage difference 128. The single AC voltage difference 128 may interact with the sensor 116 and facilitate sensing of the biomolecule 108 in the channel 106.

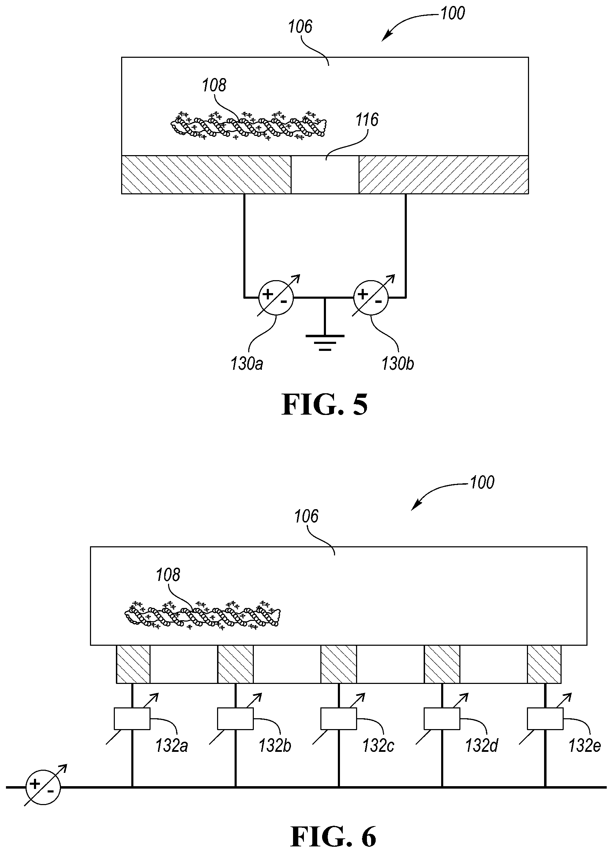

Figure 5 illustrates a device 100 having multiple voltages relative to ground 130a, 130b. The voltages 130a and 130b may interact with the sensing component 116 and facilitate sensing of the biomolecule 108 in the channel 106.

Figure 6 illustrates a device 100 having multiple voltages adjusted by resistors 132a, 132b, 132c, 132d, and 132e. The device may be a sensor. The device may sense a biomolecule. The electrodes 102a, 102b, 102c, 102d, and 102e may have a surface charge and voltage controlled by the resistors 132a, 132b, 132c, 132d and 132e and may facilitate sensing of the biomolecule 108 in the channel 106.

In various embodiments, a system for controlling the proximity of a biomolecule to an electrode is provided. The system may include a device for sensing a biomolecule. The device may include at least one electrode. The at least one electrode may be coated with a conductive low-van der Waals material to form a surface coating. The device may additionally include a channel. The channel may be a nanochannel having at least one dimension of less than 1 µm. The channel may be sized to receive a biomolecule and a solvent. The at least one electrode may be coated with the surface coating prior to introducing the biomolecule and the solvent into the channel. The at least one electrode may be a metal electrode. Alternatively, the at least one electrode may be a carbon or graphite electrode. The surface coating on the at least one electrode may be a layer of carbon or multiple layers of carbon. The carbon may be a graphene-based compound. Alternatively, the surface coating may be an MXene. The biomolecule may be a polynucleotide. For example, the biomolecule may be a DNA molecule. The system may additionally include a voltage source and a processor. The system may include a memory coupled to the processor. The memory may store executable instructions. The system may also include a controller. The executable instructions when executed by the processor may cause the controller to transmit a signal to the at least one electrode to sense the biomolecule.

In various embodiments, a system for controlling the proximity of a biomolecule to an electrode is provided. The system may include a device. The device may sense a biomolecule. The device may include at least one electrode, and a channel sized to receive the biomolecule and a solvent. The channel may be a nanochannel having at least one dimension of less than 1 µm. The device may additionally include a voltage source and a processor. The system may include a memory coupled to the processor. The memory may store executable instructions. The system may also include a controller. The executable instructions when executed by the processor may cause the controller to transmit a signal to the at least one electrode to charge the at least one electrode with a surface charge between -0.5 and +0.5 C/m2. The surface charge may modulate the distance of the biomolecule from the at least one electrode. The device may include four or more electrodes patterned in a +/-/+/- alternation. The device may include an insulator between electrodes. The signal transmitted by the controller to the at least one electrode may cause the voltage of the at least one electrode to be set to a defined value.

In various embodiments, a system for controlling the proximity of a biomolecule to an electrode is provided. The system may include a device for sensing a biomolecule including at least one electrode and a channel. The channel may be a nanochannel having at least one dimension of less than 1 µm. The biomolecule may be a polynucleotide. The device may additionally include a voltage source and a processor. The system may also include a memory coupled to the processor. The memory may store executable instructions. The system may also include a controller. The processor may execute executable instructions that may cause the controller to transmit a signal to the at least one electrode to dynamically modify a voltage of the at least one electrode by a pulse of length τ that is substantially different from the electrode sensing time τ' such that τ/τ^' is less than 0.1 or greater than 10. The controller may transmit the signal to dynamically modify a voltage of the at least one electrode to promote a physical interaction of the biomolecule with the at least one electrode while the voltage is at a first value. The controller may transmit a signal to modify the voltage of the at least one electrode to a second voltage value to promote release of the biomolecule from the physical interaction with the at least one electrode.

In various embodiments, a method for controlling the proximity of a biomolecule to an electrode is provided. The method may include providing a device including at least one electrode and a channel. The channel may be a nanochannel having at least one dimension of less than 1 µm. The at least one electrode may be a metal electrode, a carbon electrode, or a graphite electrode. The at least one electrode may be coated with a surface coating. The surface coating may be a conductive low-van der Waals material. The method may further include providing a biomolecule and a solvent to the channel of the device. The biomolecule may be a DNA molecule. The solvent may include water and salts. The method may additionally include applying a voltage to the at least one electrode to modify the surface charge of the electrode. The voltage may be static or dynamic. If the voltage is dynamic (e.g., changeable), the method may include sticking the biomolecule to the electrode for a long enough time to collect a measurement. The method may also include changing the voltage to release the biomolecule so that the flow may move the biomolecule. For example, applying a dynamic voltage to the at least one electrode may cause the biomolecule to physically interact with at least one electrode while the voltage is at a first value, and to be released from the physical interaction with the at least one electrode when the voltage is at a second value. Alternatively, the voltage fluctuation may be much faster than the measurement time, allowing an averaging effect to resist or avoid contamination of the measurement.

If there are two or more electrodes, the method may include dynamically or statically controlling the charges on each electrode to ensure a desired flow rate. The method may include patterning the two or more electrodes in a +/- alternation. For example, if there are four electrodes, the patterning may be +/-+/-.

In one or more other embodiments, a method for controlling the proximity of a biomolecule to an electrode is provided. The method may include providing a device including at least one electrode and a channel sized to receive a biomolecule and a solvent. The channel may be a nanochannel having at least one dimension of less than 1 µm. The at least one electrode may be coated with a surface coating. The surface coating may be a conductive low-van der Waals material. The method may further include providing a biomolecule and a solvent to the channel of the device. The biomolecule may be a DNA molecule. The solvent may include water and salts. The method may further include charging the electrode with a surface charge between -0.5 and +0.5 C/m2. The method may further include dynamically modifying the electrode voltage by a pulse of length τ that is substantially different from the electrode sensing time τ'. τ/τ' may be either less than 0.1 or greater than 10.

In one or more other embodiments, a method for controlling the proximity of a biomolecule to an electrode is provided. The method may include providing a device including at least one electrode and a channel. The channel may be a nanochannel having at least one dimension of less than 1 µm. The at least one electrode may be coated with a surface coating. The surface coating may be a conductive low-van der Waals material. The surface coating may be at least one layer of carbon. The surface coating may be graphene. The method may further include providing a biomolecule and a solvent to the channel of the device. The biomolecule may be a DNA molecule. The solvent may include water and salts. The surface coating, voltage, and/or salts may modulate the proximity of the biomolecule to the electrode.

In one or more other embodiments, a method for controlling the proximity of a biomolecule to an electrode is provided. The method may include providing a device including at least one electrode and a channel. The channel may be a nanochannel having at least one dimension of less than 1 µm. The method may further include charging the at least one electrode with a surface charge between -0.5 and +0.5 C/m2. The device may include a plurality of electrodes. The plurality of electrodes may have a pattern of alternating different charges. The method may further include providing a biomolecule and a solvent to the channel of the device. The biomolecule may be a DNA molecule. The solvent may include water and salts.

In other embodiments, a method for controlling the proximity of a biomolecule to an electrode is provided. The method may include providing a device including at least one electrode and a channel. The channel may be a nanochannel having at least one dimension of less than 1 µm. The method may further include dynamically modifying the electrode voltage by a pulse of length τ that is substantially different from the electrode sensing time τ'. *τ*/*τ*' may be either less than 0.1 or greater than 10. The method may further include providing a biomolecule and a solvent to the channel of the device. The biomolecule may be a DNA molecule. The solvent may include water and salts.

### Example: Simulating the interaction of a DNA molecule with a metal electrode

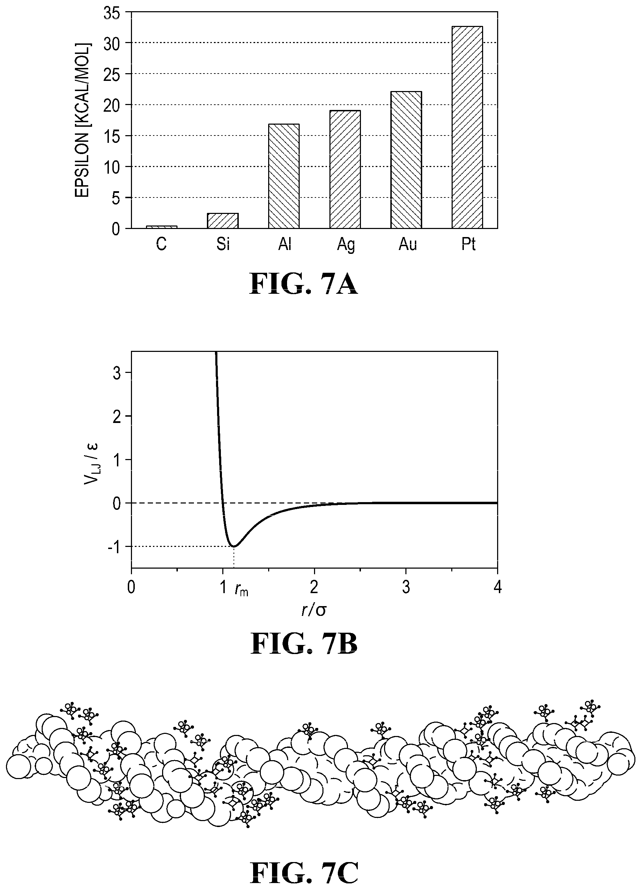

Figures 7A and 7B are graphs illustrating data from a simulation of the interaction of a DNA molecule with a metal electrode, using a molecular dynamics simulation. The van der Waals (vdW) interaction depends on the electrode material, as shown in Figures 7A and 7B. Figure 7C is an image of the interaction of the DNA with the metal electrode using the molecular dynamics simulation.

The electrode additionally has a surface charge that may be varied between -0.5 C/m2 to +0.5 C/m2. Water and salt are included in the simulation as surrounding the DNA. Using an umbrella sampling technique at room temperature, the energy vs. distance can be measured. Using a biased molecular dynamics simulation, the DNA molecule was positioned at different distances from the electrode surface and simulation data were collected in a technique called umbrella sampling. The results were then processed to extract the energy vs separation distance plots to assess how strongly the DNA strand was impacted by the charging states and the material of the electrode. Simulations are done at room temperature conditions. As shown by the van der Waals interactions, the two extrema are carbon and platinum, so the energy vs. distance was measured for a carbon electrode and a platinum electrode.

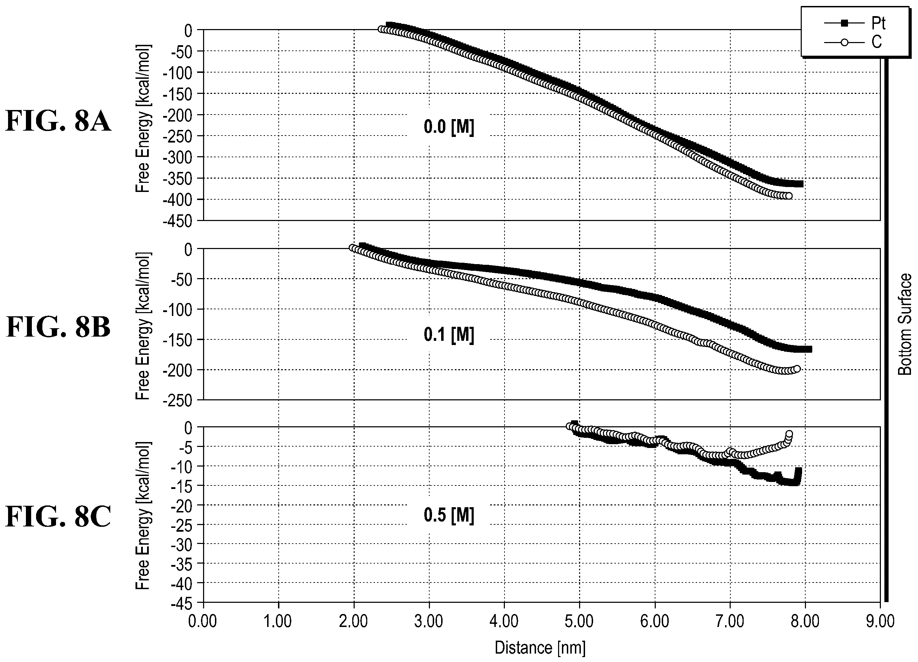

The effects of the salt concentration on the energy vs. distance were measured. Figure 8 illustrates the free energy of interaction at different salt concentrations and the distance between the DNA center and the electrode surface. It is found that the high-vdW Pt material has a much stickier behavior (lower free energy) compared to the low-vdW carbon material. When combined with an appropriate amount of salt, the overall equilibrium distance between the molecule center and the electrode can be tuned.

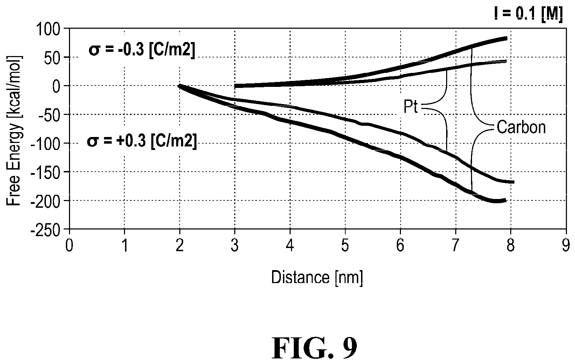

Figure 9 illustrates the dependencies of different surface charging states at a fixed salt concentration. It is found that even when using platinum, modulating the surface charging state can substantially change the energy and force of the molecule exerted by the electrode. It is well-known that a voltage on an electrode will vary the surface charge accordingly. In one or more embodiments, modulating the electrode surface material, salt concentration, and surface charge facilitates the modulation of the dependence of the free energy on the distance from the electrode.

While exemplary embodiments are described above, it is not intended that these embodiments describe all possible forms encompassed by the claims. The words used in the specification are words of description rather than limitation, and it is understood that various changes can be made without departing from the spirit and scope of the disclosure. As previously described, the features of various embodiments can be combined to form further embodiments of the invention that may not be explicitly described or illustrated. While various embodiments could have been described as providing advantages or being preferred over other embodiments or prior art implementations with respect to one or more desired characteristics, those of ordinary skill in the art recognize that one or more features or characteristics can be compromised to achieve desired overall system attributes, which depend on the specific application and implementation. These attributes can include, but are not limited to cost, strength, durability, life cycle cost, marketability, appearance, packaging, size, serviceability, weight, manufacturability, ease of assembly, etc. As such, to the extent any embodiments are described as less desirable than other embodiments or prior art implementations with respect to one or more characteristics, these embodiments are not outside the scope of the disclosure and can be desirable for particular applications.

## Claims

1. A system for controlling the proximity of a biomolecule to an electrode comprising:
a device for sensing a biomolecule including:
at least one electrode, wherein the at least one electrode is coated with a conductive low-van der Waals material to form a surface coating, and
a channel sized to receive the biomolecule and a solvent, wherein the surface coating of the electrode is configured to modulate a distance of the biomolecule from the at least one electrode in the channel;
a voltage source; and
a processor, a memory coupled to the processor and storing executable instructions, and a controller, wherein the executable instructions, when executed by the processor, cause the controller to transmit a signal to the at least one electrode to sense the biomolecule.

2. The system of claim 1, wherein the at least one electrode includes a metal electrode.

3. The system of claim 2, wherein the at least one electrode includes a platinum electrode.

4. The system of claim 1, wherein the at least one electrode includes a carbon electrode.

5. The system of claim 1, wherein the surface coating is a layer of carbon.

6. The system of claim 5, wherein the carbon of the layer includes a graphene-based compound.

7. The system of claim 1, wherein the surface coating is an MXene.

8. The system of claim 1, wherein the biomolecule is a polynucleotide.

9. The system of claim 1, wherein the channel is a nanochannel having at least one dimension of less than 1 µm.

10. A system for controlling the proximity of a biomolecule to an electrode comprising:
a device for sensing a biomolecule including:
at least one electrode and a channel sized to receive the biomolecule and a solvent;
a voltage source; and
a processor, a memory coupled to the processor and storing executable instructions, and a controller, wherein the executable instructions, when executed by the processor, cause the controller to transmit a signal to the at least one electrode to charge the at least one electrode with a surface charge between -0.5 and +0.5 C/m², wherein the surface charge modulates a distance of the biomolecule from the at least one electrode.

11. The system of claim 10, wherein the at least one electrode includes two or more electrodes patterned in a +/- alternation.

12. The system of claim 10, wherein the device further includes an insulator between electrodes.

13. The system of claim 10, wherein the signal transmitted by the controller to the at least one electrode causes the voltage of the at least one electrode to be set to a defined value.

14. A system for controlling the proximity of a biomolecule to an electrode comprising:
a device for sensing a biomolecule including:
at least one electrode and a channel sized to receive the biomolecule and a solvent;
a voltage source; and
a processor, a memory coupled to the processor and storing executable instructions, and a controller, wherein the executable instructions, when executed by the processor, cause the controller to transmit a signal to the at least one electrode to dynamically modify a voltage of the at least one electrode by a pulse of length τ that is substantially different from the electrode sensing time τ' such that *τ*/*τ'* is less than 0.1 or greater than 10.

15. The system of claim 14, wherein the channel is a nanochannel having a diameter of less than 1 µm.

16. The system of claim 14, wherein the biomolecule is a polynucleotide.

17. The system of claim 14, wherein the controller transmits the signal to dynamically modify a voltage of the at least one electrode, wherein the biomolecule physically interacts with the at least one electrode while the voltage is at a first value, and wherein the biomolecule is released from the physical interaction with the at least one electrode at a second value of voltage.

18. A method for controlling the proximity of a biomolecule to an electrode comprising:
providing a device including at least one electrode and a channel;
providing a biomolecule and a solvent to the channel of the device; and
modulating a distance of the biomolecule from the at least one electrode by one of:
(i) coating the at least one electrode with a surface coating prior to providing the biomolecule and the solvent to the channel;
(ii) applying a voltage to the at least one electrode to modify the surface charge of the electrode; or
(iii) dynamically modifying a voltage of the at least one electrode.

19. The method of claim 18, wherein the surface charge is between -0.5 and +0.5 C/m².

20. The method of claim 18, wherein the voltage of the at least one electrode is modified by a pulse of length τ that is substantially different from the electrode sensing time τ' such that τ/τ' is less than 0.1 or greater than 10.
